# EUROPEAN PATENT SPECIFICATION

(11) **EP 0 190 018 B1**
(45) Date of publication and mention of the grant of the patent: **07.04.1993**
(21) Application number: 86300486.7
(22) Date of filing: 24.01.1986
(51) Int. Cl.: A61K 37/02

(54) **Use of transforming growth factor to promote wound healing and composition therefor**
Verwendung des Transformationswachstumsfaktors zur Förderung der Wundheilung und Zusammensetzung dafür
Application du facteur de transformation de croissance pour favoriser la guérison des plaies et composition à cette fin

(30) Priority: 29.01.1985 US 695983
(43) Date of publication of application: 06.08.1986
(73) Proprietor: ONCOGEN, Seattle, WA 98121 (US)
(72) Inventor: Todaro, George J., Seattle Washington 98112 (US); Schultz, Gregory L., Louisville Kentucky (US); Eiferman, Richard, Kentucky 40202 (US)
(74) Representative: Goldin, Douglas Michael

(56) References cited:
- EP-A- 0 105 014
- EP-A- 0 132 021
- EP-A- 0 154 434
- PROCEEDINGS OF THE NATIONAL ACADEMY OF SCIENCES USA, vol. 80, June 1983, pages 3676-3680; C.A. FROLIK et al.: "Purification and initial characterization of a type Beta-transforming growth factor from human placenta"
- SCIENCE, vol. 223, 9th March 1984, pages 1079-1082; H. MARQUARDT et al.: "Rat transforming growth factor type 1: Structure and relation to epidermal growth factor"

## Description

Epithelial and stromal wounds can result from a number of causes, such as burns, abrasions, cuts, surgical procedures and other injuries. Long healing times for such wounds are a particular problem, and can result in complications such as infection, pain, and delayed recovery.

The healing of corneal stromal wounds is a major problem. For example, stromal incisions following corneal transplantation can require from 6 to 9 months to heal, during which time the patient suffers from poor visual acuity and increased risk of infection in the wound. No agent for the promotion of wound healing, particularly corneal wound healing, is presently available for routine clinical use.

It would thus be desirable to provide a method and agent for treating epithelial and stromal corneal wounds to promote their rapid healing. In particular, it would be desirable to provide such wound healing compositions in large quantities and in formulations suitable for clinical treatment.

Various agents have been studied for possible use in the treatment of corneal stromal incisions. Parenterally administered ascorbate (vitamin C) was applied topically to full thickness incisions in rabbit corneas, but failed to provide increased tensile strength during the healing. Pfister et al. (1981) Invest. Ophthalmol. 21:80-86. Topical administration of mesodermal growth factor, a partially purified protein prepared from the submandibular glands of mice, appeared to activate stromal fibroblasts in the region of partial thickness incisions in rabbit corneas. Rich et al. (1979) Arch. Ophthalmol. 97:1326-1330. Epidermal growth factor purified from mouse submandibular gland was shown to increase the tensile strength of full thickness incisions in rabbit corneas. Brightwell et al., in an oral presentation at the 13th Biennial Corneal Research Conference, Boston, Massachusetts, September 23, 1983. Various studies have examined the use of mouse epidermal growth factor in treating cutaneous wounds. Greaves (1980) Clin. Exp. Dermatol. 5:101-103, applied mouse EGF on blister wounds on human subjects. The mEGF in saline solution was applied once daily until the wounds healed. No acceleration in the growth of the epidermal layer was observed. Topical application of mouse EGF to open wounds in mice has been found to promote healing in various degrees. See, e.g., Niall et al. (1982) J. Surg. Res. 33:164-169; Thornton et al. (1981) Burns 8:156-160.

The hypothesis that some transformed cells produce endogenous transforming growth factors has been supported by isolation of peptide factors from transformed cells. Delarco and Todaro (1978) Proc. Natl. Acad. Sci. USA 75:4001-4005; Roberts et al. (1982) Nature 295:417-419; Ozanne et al. (1982) Fedn. Proc. 41:3004-3007; Anzano et al. (1982) Cancer Res. 42:4776-4778; and Marquardt et al. (1984) Science 223:1079-1082. The preparation of synthetic transforming growth factor is reported in Tam et al. (1984) Nature 309:376-378.

EP-A-154 434, published on 11 September 1985 discloses the use of recombinant DNA technology to produce TGF-α. The first priority document of this application discloses that TGF-α, in combination with TGF-β, "could find use" for example in the therapeutic treatment of human beings.

EP-A-105 014 discloses a composition for the promotion of cell proliferation and tissue repair in animals having as active ingredients a TGF-β which is activated by either a TGF-α or EGF or both.

EP-A-132 021 discloses TGF polypeptides which exhibit cell growth promoting properties.

The present invention relates to compositions which have utility in methods for treating epithelial and stromal wounds to promote their rapid healing. The methods utilize treatment compositions including a polypeptide having mitogenic activity analogous to that of naturally-occurring transforming growth factor-α (TGF-α) obtained from transformed cells, which polypeptide is substantially free from contaminants which are naturally present with TGF-α. The compositions are effective in promoting the healing of various injuries, including cutaneous wounds, corneal wounds, and injuries to the hollow, epithelial-lined organs of the body. The compositions are applied topically to the affected area in physiologically acceptable carriers or bases.

The present invention thus provides a composition comprising transforming growth factor-α (TGF-α) substantially free from naturally occurring contaminants and from transforming growth factor-β in a physiologically-acceptable carrier characterized in that the TGF-α is at a concentration from 50 ng/ml to 10 mg/ml. In a further aspect, the invention provides such compositions for use in the treatment and promotion of healing of epithelial, stromal and corneal wounds, and the use of such compositions for the manufacture of a medicament for the treatment and promotion of healing of such wounds.

Thus, the present invention can be used in a method for treating wounds as mentioned above, such as cutaneous wounds, and various other epithelial and stromal disruptions, such as chronic ulcers, burns, surgical incisions, traumatic wounds, and injuries to the hollow, epithelial-lined organs, such as the esophagus, stomach, large and small bowels, mouth, genital, and urinary tract. The method relies on the topical application of a treatment composition including a polypeptide having an amino acid sequence and mitogenic activity analogous to that of transforming growth factor (TGF) obtained from transformed cells in a physiologically-acceptable carrier. The polypeptide may be obtained by purification from its natural source, or by synthetic means such as solid-phase synthesis techniques or recombinant DNA techniques. By topically applying the treatment composition to the wounds over a three day period, it was found that the rate of the healing process was substantially increased.

Transforming growth factor (TGF) is a 50 amino acid polypeptide produced by various avian and mammalian cells which have been transformed by single-stranded RNA retroviruses, certain human tumor cell lines, and a limited number of chemically-transformed animal cell lines. TGF exists in two forms, designated TGF-α and TGF-β, and the physiological role for both forms is unknown at present. The amino acid sequence of TGF-α is reported in Marquardt et al. (1984) Science 223:1079-1082.

As used hereinafter and in the claims, transforming growth factor-α or TGF-α will refer to a polypeptide product which displays biological activity, particularly mitogenic activity (i.e., the growth promotion of epithelial and stromal cells), analogous to that of natural transforming growth factor-α protein as measured in recognized bioassays, such as those reported by Cifone and Fidler (1980) Proc. Natl. Acad. Sci. USA 77:1039-1043, and DeLarco and Todaro (1978) Proc. Natl. Acad. Sci. USA 75:4001-4005. The polypeptide product will have an amino acid sequence which is the same or substantially the same as the natural protein, differing by no more than five amino acids, usually differing by three or fewer amino acids. For the most part, the TGF amino acid sequence will differ, if at all, by substitutions among the non-polar amino acids, i.e., aliphatic and aromatic amino acids. The deviations from the natural amino acid sequence will not significantly adversely affect the mitogenic activity of the polypeptide product or its ability to promote epithelial and stromal healing. As stated hereinbefore, the TGF polypeptide may be obtained from a natural source by suitable separation techniques, and purified to remove other proteins and substances naturally present in the source. Alternatively, the polypeptide may be produced by a synthetic technique, such as solid-phase synthesis of the polypeptide, or by expression of a TGF-α gene in a suitable microorganism host. Such synthetic procedures produce a product which is substantially free from naturally-occurring contaminants.

The TGF polypeptide may be isolated and purified from a natural source as follows. Suitable natural sources for the polypeptide include various animal cells transformed by oncogenic single-stranded RNA retroviruses such as Maloney mouse sarcoma virus (MSV), Abelson murine leukemia (FeSV), Snyder-Theilen FeSV and McDonough FeSV; certain human tumor cell lines such as human metastatic melanoma cell lines A673 and A2058, as well as a limited number of chemically transformed animal cell lines. The transformed or tumor cell lines are grown to confluence in serum-supplemented medium, and then transferred to serum-free medium to produce a conditioned medium. The serum-free conditioned medium may then be collected periodically, e.g., once a day, and clarified by continuous flow centrifugation. After concentration, the TGF-α may be separated from the conditioned media by gel chromatography, affinity chromatography, or the like.

Alternatively, a TGF polypeptide may be prepared by conventional solid-phase synthesis techniques, such as those described by Merrifield (1963) J. Am. Chem. Soc. 85:2149-2156. The amino acid sequence may be based on the known sequences for human and rat TGF-αas follows: 
hTGF-α
ValValSerHisPheAsnAspCysProAspSerHisThrGlnPheCys
PheHisGlyThrCysArgPheLeuValGlnGluAspLysProAlaCysVal
CysHisSerGlyTyrValGlyAlaArgCysGluHisAlaAspLeuLeuAla
rTGF-α
ValValSerHisPheAsnLysCysProAspSerHisThrGlnTyrCys
PheHisGlyThrLysArgPheLeuValGlnGluGluLysProAlaCysVal
CysHisSerGlyTyrValGlyValArgCysGluHisAlaAspLeuLeuAla
A specific protocol for synthesizing the rTGF-α polypeptide is set forth in the Experimental section hereinafter.

The TGF polypeptides may also be produced by expression of a TGF-α gene in a suitable microorganism host. The TGF gene may be viral DNA, cDNA, synthetic DNA, or a combination thereof, e.g., synthetic DNA may be combined with the cDNA to complete the TGF-α gene. Conveniently, the present invention will utilize a synthetic DNA sequence based on the amino acid sequence(s) of TGF-α (above). A suitable synthetic DNA sequence may be prepared by synthesizing single-stranded DNA fragments by well known techniques, e.g., the phosphoramidite method described by Beaucage and Carruthers (1981) Tetrahedron Lett. 22:1859-1862. Complementary strands may be annealed under appropriate conditions to provide the desired double-stranded synthetic gene.

For expression, the TGF-α gene may be incorporated in an extrachromosomal element including a replication system recognized by a desired host, typically E. coli or yeast, and transcriptional and translational regulatory control sequences which control the expression of the TGF-α gene. The extrachromosomal element may include a number of other features, such as selectable markers, which facilitate manipulation of the extrachromosomal element.

After a suitable extrachromosomal element has been prepared, it can be used to transform an appropriate host, and the polypeptide product expressed in that host recovered and purified for use in the compositions of the present invention. Purification of the polypeptide product may be accomplished in a variety of ways, including ion exchange chromatography, high performance liquid chromatography, and the like. It is desired that the TGF-α be greater than 70% pure, preferably at least 80% pure, more preferably at least 90% pure.

The compositions of the present invention will be useful for treating a wide variety of wounds including substantially all cutaneous wounds, corneal wounds, and injuries to the epithelial-lined hollow organs of the body. Wounds suitable for treatment include those resulting from trauma such as burns, abrasions and cuts, as well as from surgical procedures such as surgical incisions and skin grafting. Other conditions suitable for treatment with the compositions of the present invention include chronic conditions, such as chronic ulcers, diabetic ulcers, and other non-healing (trophic) conditions. Compositions will find particular use in treating corneal and scleral wounds, including wounds which affect the epithelial layer, stromal layer and endothelial layer of the cornea. Treatment according to the present invention will promote cell division of the endothelial layer of the cornea.

The polypeptides will be incorporated in physiologically-acceptable carriers for application to the affected area. The nature of the carriers may vary widely and will depend on the intended location of application. For application to the skin, a cream or ointment base is usually preferred, suitable bases include lanolin, Silvadene® (Marion) (particularly for the treatment of burns), or Aquaphor® (Duke Laboratories, South Norwalk, Connecticut). If desired, it will be possible to incorporate the TGF-carrier compositions in bandages and other wound dressings to provide for continuous exposure of the wound to the TGF. Aerosol applicators may also find use.

For corneal treatment, the carrier will be suitable for application to the eyes. Suitable carriers include ointments, sterile saline solutions, isotonic saline solutions, such as Sorbi-care™ (Allergan Pharmaceutical), or Neodecadron® (Merck, Sharp and Dohme). A suitable ointment base is sold under the tradename Lacrilube®. The occular carriers will normally include preservatives, such as benzalkonium chloride and edetate disodium, unless they are formulated immediately prior to application.

Often, it may be desirable to incorporate the TGF-α in liposomes to provide for release of the TGF-α over an extended period, typically from 24 to 48 hours. Such incorporation may be particularly desirable when the TGF-α is incorporated into a wound dressing, as described above.

The concentration of the polypeptide in the composition is from 50 µg/ml to 10 mg/ml, usually the polypeptide will be present between 10µg/ml and 10mg/ml. The compositions will be applied topically to the affected area, typically as eye drops to the eye or as creams, ointments or lotions to the skin. In the case of eyes, frequent treatment is desirable, usually being applied at intervals of 4 hours or less. On the skin, it is desirable to continually maintain the treatment composition on the affected area during the healing, with applications of the treatment composition from two to four times a day or more frequently.

Optionally, the compositions of the present invention may be combined with effective amounts of anesthetics, antibiotics, antiseptics, and other drugs, typically present at about 0.001% to 2% by weight.

The following experimental results are offered by way of example.

### EXPERIMENTAL

### Materials and Methods

### 1. Preparation of rTGF-α polypeptide

The TGF-α polypeptide was synthesized based on the amino acid sequence reported by Marquardt et al., supra. for TGF purified from the conditioned medium of Fisher rat embryo fibroblasts transformed by feline sarcoma virus. The sequence was as follows: 
H-Val-Val-Ser-His-Phe-Asn-Lys-Cys-Pro-Asp-Ser-
His-Thr-Gln-Tyr-Cys-Phe-His-Gly-Thr-Cys-Arg-Phe-
Leu-Val-Gln-Glu-Glu-Lys-Pro-Ala-Cys-Val-Cys-His-
Ser-Gly-Tyr-Val-Gly-Val-Arg-Cys-Glu-His-Ala-Asp-
Leu-Leu-Ala-OH
The chemical synthesis of rTGF-α was performed manually by the stepwise solid-phase approach, following the general principles described by Merrifield, supra., employing the differential acid-labile protecting group strategy, using the conventional combination of t-butyloxycarbonyl for the N^{α}-amino terminus and benzyl alcohol derivatives for the side chains. An improved, more acid-stable benzyl ester linkage that anchored protected amino acids to the polymeric support was used to minimize loss of peptides during the repeated acid treatments. Mitchell et al. (1976) J. Am. Chem. Soc. 98:7357-7362. Complete deprotection and removal of peptide from the resin was achieved by the low-high HF method (Tam et al. (1982) Tetrahedron Lett. 23:4435-4438, and Tam et al. (1983) J. Am. Chem. Soc. 105:6442-6454), which removed benzyl protecting groups by the S_{N}2 mechanism in dilute HF solution; this minimizes the serious side reactions due to carbocations generated in the conventional S_{N}1 deprotection method. It also minimizes the cystinyl side reactions that can hamper the synthesis of proteins containing multiple disulphide linkages.

After HF treatment and before any purification, the crude and reduced synthetic rTGF-α was oxidized and regenerated by the mixed disulphide method in the presence of a combination of reduced and oxidized glutathione. Ahmed et al. (1975) J. Biol. Chem. 250:8477-8482. This avoided the formation of polymeric materials during purification. The regenerated, crude rTGF-α contained 40-50% of EGF radioreceptor and tyrosine-specific protein kinase activities when compared with natural rTGF-α. Crude synthetic rTGF-α was purified to homogeneity in three steps: (1) gel filtration on a BioGel P-10 column, (2) ion-exchange chromatography on a CM-Sephadex column and (3) preparative HPLC on a C₁₈ reverse-phase column. Overall yield, based on starting loading of Ala to resin, was 31%.

### 2. Preparation of treatment formulation

The purified rTGF-α polypeptide was combined with isotonic (285 m osmoles) sterile phosphate buffered saline (pH 7.4) at a concentration of 50ng/ml.

### 3. Corneal Stromal Incisions

Totally penetrating incisions 5mm in length which extended into the anterior chamber along their entire length were made in the center corneas of adult female Macaca fasicularis primates. The right eyes served as controls and were treated three times each day with two drops of isotonic (285 m osmoles) sterile phosphate buffered saline (pH 7.4) without rTGF-α. The left eyes were treated on the same schedule with the treatment formulation described above. After three days of treatment, the strength of the wounds was quantitatively measured by inserting a small bore needle (25 gauge) into the anterior chamber through the limbus of the cornea. The needle was connected to an aneroid manometer, and the pressure was slowly and steadily increased until the wounds first began to leak, and then burst. This procedure is described in detail by Weene (1983) Anal. Ophthalmol. 15:438.

### Results

The results shown in Table 1 demonstrate that the bursting strength of TGF-α treated corneas is significantly stronger than the saline treated control cornea.

**Table 1**

| | mm of Hg | | | |
|---|---|---|---|---|
| | Control Right Eye | | TGF-treated Left Eye | |
| | Leak | Burst | Leak | Burst |
| Monkey P-21 | 25 | 30* | 210 | 225* |
| Monkey P-20 | 35 | 90* | 155 | >300* |

| | | | | |
|---|---|---|---|---|
| *t = 40.0, p < 0.025, paired T-test. | | | | |

According to the present invention, a wound treatment composition is provided which promotes the rapid healing of epithelial and stromal wounds, including cutaneous wounds, corneal wounds, and wounds to the hollow, epithelial-lined body organs. The compositions include a polypeptide product having an amino acid sequence and mitogenic activity corresponding to that of natural transforming growth factor. The compositions are found to greatly enhance the healing of full thickness incisions in the cornea.

## Claims (Claims for the following Contracting State(s): BE, CH, DE, FR, GB, IT, LU, NE, SE)

1. A composition for use in the treatment of epithelial or stromal wounds in the human or animal body comprising transforming growth factor-α (TGF-α) substantially free from naturally occurring contaminants and from transforming growth factor-β in a physiologically-acceptable carrier characterized in that the TGF-α is at a concentration from 50 ng/ml to 10 mg/ml.

2. A composition according to claim 1 for use in the promotion of the healing of wounds in the cornea.

3. A composition according to claim 1 or 2 wherein the carrier is an ointment or a sterile saline solution.

4. A composition according to any of claims 1 to 3 wherein the TGF-α is rTGF-α.

5. A composition according to any of claims 1 to 4 wherein the TGF-α is synthesized in vitro.

6. A composition according to any of claims 1 to 5, wherein the TGF-α is prepared by solid-phase synthesis.

7. Use of a composition comprising transforming growth factor-α (TGF-α) substantially free of naturally occurring contaminants and from transforming growth factor-β in physiologically acceptable carrier characterised in that the TGF-α is at a concentration of from 50ng/ml to 10mg/ml for the manufacture of a medicament for promotion of healing of wounds in the cornea.

## Claims (Claims for the following Contracting State(s): AT)

1. A method for preparing a composition comprising transforming growth factor-α (TGF-α) substantially free from naturally occurring contaminants and from transforming growth factor-β for use in the treatment of epithelial or stromal or corneal wounds in the human or animal body comprising admixing the said TGF-α with a physiologically-acceptable carrier at a concentration of from 50 ng/ml to 10 mg/ml.

2. A method according to claim 1 wherein the carrier is an ointment, or a sterile saline solution.

3. A method according to claim 1 or 2, wherein the TGF-α is rTGF-α.

4. A method according to any of the preceding claims, wherein the TGF-α is synthesized in vitro.

5. A method according to claim 4, wherein the TGF-α is prepared by solid-phase synthesis.

6. A composition for use in the treatment of epithelial or stromal wounds in the human or animal body comprising transforming growth factor-α (TGF-α) substantially free from naturally occurring contaminants and from transforming growth factor-β in a physiologically-acceptable carrier characterized in that the TGF-α is at a concentration from 50 ng/ml to 10 mg/ml.

7. A composition according to claim 6 for use in the promotion of the healing of wounds in the cornea.

8. A composition according to claim 6 or 7 wherein the carrier is an ointment or a sterile saline solution.

9. A composition according to any of claims 6 to 8 wherein the TGF-α is rTGF-α.

10. A composition according to any of claims 6 to 9 wherein the TGF-α is synthesized in vitro.

11. A composition according to any of claims 1 to 10, wherein the TGF-α is prepared by solid-phase synthesis.

12. Use of a composition comprising transforming growth factor-α (TGF-α) substantially free of naturally occurring contaminants and from transforming growth factor-β in physiologically acceptable carrier characterised in that the TGF-α is at a concentration of from 50ng/ml to 10mg/ml for the manufacture of a medicament for promotion of healing of wounds in the cornea.

## Patentansprüche (Patentansprüche für folgende(n) Vertragsstaat(en): BE, CH, DE, FR, GB, IT, LU, NL, SE)

1. Zusammensetzung zur Verwendung bei der Behandlung von Epithel- oder Stromawunden im menschlichen oder tierischen Körper, umfassend den transformierenden Wachstumsfaktor-α (TGF-α), der im wesentlichen frei von natürlich auftretenden Verunreinigungen und von transformierendem Wachstumsfaktor-β ist, in einem physiologisch verträglichen Träger, dadurch gekennzeichnet, daß der TGF-α in einer Konzentration von 50 ng/ml bis 10 mg/ml vorliegt.

2. Zusammensetzung nach Anspruch 1 zur Verwendung bei der Förderung der Heilung von Wunden in der Cornea.

3. Zusammensetzung nach Anspruch 1 oder 2, worin der Träger eine Salbe oder eine sterile Salzlösung ist.

4. Zusammensetzung nach einem der Ansprüche 1 bis 3, worin der TGF-α rTGF-α ist.

5. Zusammensetzung nach einem der Ansprüche 1 bis 4, worin der TGF-α in vitro synthetisiert ist.

6. Zusammensetzung nach einem der Ansprüche 1 bis 5, worin der TGF-α durch Festphasensynthese hergestellt ist.

7. Verwendung einer Zusammensetzung, die den transformierenden Wachstumsfaktor-α (TGF-α) umfaßt, der im wesentlichen frei von natürlich auftretenden Verunreinigungen und von transformierendem Wachstumsfaktor-β ist, in einem physiologisch verträglichen Träger, dadurch gekennzeichnet, daß der TGF-α in einer Konzentration von 50 ng/ml bis 10 mg/ml vorliegt, zur Herstellung eines Medikamentes zur Förderung der Heilung von Wunden in der Cornea.

## Patentansprüche (Patentansprüche für folgende(n) Vertragsstaat(en): AT)

1. Verfahren zur Herstellung einer Zusammensetzung, umfassend den transformierenden Wachstumsfaktor-α (TGF-α), der im wesentlichen frei von natürlich auftretenden Verunreinigungen und von transformierendem Wachstumsfaktor-β ist, zur Verwendung bei der Behandlung von Epithel- oder Stroma- oder Corneawunden im menschlichen oder tierischen Körper, umfassend das Mischen dieses TGF-α mit einem physiologisch verträglichen Träger bei einer Konzentration von 50 ng/ml bis 10 mg/ml.

2. Verfahren nach Anspruch 1, worin der Träger eine Salbe oder eine sterile Salzlösung ist.

3. Verfahren nach Anspruch 1 oder 2, worin der TGF-α rTGF-α ist.

4. Verfahren nach einem der vorhergehenden Ansprüche, worin der TGF-α in vitro synthetisiert wird.

5. Verfahren nach Anspruch 4, worin der TGF-α durch Festphasensynthese hergestellt wird.

6. Zusammensetzung zur Verwendung bei der Behandlung Epithel- oder Stromawunden im menschlichen oder tierischen Körper, umfassend den transformierenden Wachstumsfaktor-α (TGF-α), der im wesentlichen frei von natürlich auftretenden Verunreinigungen und von transformierendem Wachstumsfaktor-β ist, in einem physiologisch verträglichen Träger, dadurch gekennzeichnet, daß der TGF-α bei einer Konzentration von 50 ng/ml bis 10 mg/ml vorliegt.

7. Zusammensetzung nach Anspruch 6 zur Verwendung bei der Förderung der Heilung von Wunden in der Cornea.

8. Zusammensetzung nach Anspruch 6 oder 7, worin der Träger eine Salbe oder eine sterile Salzlösung ist.

9. Zusammensetzung nach einem der Ansprüche 6 bis 8, worin der TGF-α rTGF-α ist.

10. Zusammensetzung nach einem der Ansprüche 6 bis 9, worin der TGF-α in vitro synthetisiert ist.

11. Zusammensetzung nach einem der Ansprüche 1 bis 10, worin der TGF-α durch Festphasensynthese hergestellt ist.

12. Verwendung einer Zusammensetzung, umfassend den transformierenden Wachstumsfaktor-α (TGF-α), der im wesentlichen frei von natürlich auftretenden Verunreinigungen und von transformierendem Wachstumsfaktor-β ist, in einem physiologisch verträglichen Träger, dadurch gekennzeichnet, daß der TGF-α in einer Konzentration von 50 ng/ml bis 10 mg/ml vorliegt, zur Herstellung eines Medikamentes zur Förderung der Heilung von Wunden in der Cornea.

## Revendications (Revendications pour l'(les) Etat(s) contractant(s) suivant(s): BE, CH, DE, FR, GB, IT, LU, NE, SE)

1. Composition destinée au traitement des blessures épithéliales ou stromales du corps humain ou animal comprenant le facteur de croissance transformant alpha (TGF-α) sensiblement exempt d'impuretés naturelles et de facteur de croissance transformant bêta, dans un véhicule physiologiquement acceptable, caractérisée en ce que le TGF-α est à une concentration de 50 ng/ml à 19 mg/ml.

2. Composition selon la revendication 1 destinée à favoriser la cicatrisation des blessures de la cornée.

3. Composition selon la revendication 1 ou 2 dans laquelle le véhicule est un onguent ou une solution saline stérile.

4. Composition selon l'une quelconque des revendications 1 à 3 dans laquelle le TGF-α est le rTGF-α.

5. Composition selon l'une quelconque des revendications 1 à 4 dans laquelle le TGF-α est synthétisé in vitro.

6. Composition selon l'une quelconque des revendications 1 à 5, dans laquelle le TGF-α est préparé par synthèse en phase solide.

7. Utilisation d'une composition comprenant le facteur de croissance transformant alpha (TGF-α) sensiblement dépourvu d'impuretés naturelles et de facteur de croissance transformant bêta dans un véhicule physiologiquement acceptable, caractérisée en ce que le TGF-α est à une concentration de 50 ng/ml à 10 mg/ml pour la préparation d'un médicament destiné à favoriser la cicatrisation des blessures de la cornée.

## Revendications (Revendications pour l'(les) Etat(s) contractant(s) suivant(s): AT)

1. Procédé de préparation d'une composition comprenant le facteur de croissance transformant alpha (TGF-α) sensiblement exempt d'impuretés naturelles et de facteur de croissance transformant bêta destinée au traitement des maladies épithéliales, stromales ou cornéales du corps humain ou animal, dans lequel on mélange ledit TGF-α avec un véhicule physiologiquement acceptable à une concentration de 50 ng/ml à 10 mg/ml.

2. Procédé selon la revendication 1 dans lequel le véhicule est un onguent ou une solution saline stérile.

3. Procédé selon la revendication 1 ou 2 dans lequel le TGF-α est le rTGF-α.

4. Procédé selon l'une quelconque des revendications précédentes, dans lequel le TGF-α est synthétisé in vitro.

5. Procédé selon la revendication 4, dans lequel on prépare le TGF-α par synthèse en phase solide.

6. Composition destinée au traitement des blessures épithéliales ou stromales du corps humain ou animal comprenant le facteur de croissance transformant alpha (TGF-α) sensiblement exempt d'impuretés naturelles et de facteur de croissance transformant bêta dans un véhicule physiologiquement acceptable, caractérisée en ce que le TGF-α est à une concentration de 50 ng/ml à 10 mg/ml.

7. Composition selon la revendication 6 destinée à favoriser la cicatrisation des blessures de la cornée.

8. Composition selon la revendication 6 ou 7 dans laquelle le véhicule est un onguent ou une solution saline stérile.

9. Composition selon l'une quelconque des revendications 6 à 8 dans laquelle le TGF-α est le rTGF-α.

10. Composition selon l'une quelconque des revendications 6 à 9 dans laquelle de TGF-α est synthétisé in vitro.

11. Composition selon l'une quelconque des revendications 1 à 10, dans laquelle on prépare le TGF-α par synthèse en phase solide.

12. Utilisation d'une composition comprenant le facteur de croissance transformant alpha (TGF-α) sensiblement exempt d'impuretés naturelles et de facteur de croissance transformant bêta dans un véhicule physiologiquement acceptable, caractériseée en ce que le TGF-α est à une concentration de 50 ng/ml à 10 mg/ml pour la préparation d'un médicament destinée à favoriser la cicatrisation des blessures de la cornée.
